# EUROPEAN PATENT APPLICATION

(11) **EP 3 205 341 A1**
(43) Date of publication of application: **16.08.2017**
(21) Application number: 15848668.8
(22) Date of filing: 15.09.2015
(51) Int. Cl.: A61K 31/192, A61K 9/70, A61K 47/14, A61K 47/44, A61P 29/00

(54) **EXTERNAL PREPARATION COMPOSITION CONTAINING S-FLURBIPROFEN**

(30) Priority: 07.10.2014 JP 2014206781
(71) Applicant: Taisho Pharmaceutical Co., Ltd., Tokyo 170-8633 (JP); Tokuhon Corporation, Tokyo 171-0033 (JP)
(72) Inventor: UCHIKAWA, Yasumasa, Saitama 345-0824 (JP); SATO, Takayuki, Tokyo 170-8633 (JP); MATSUKI, Kota, Tokyo 170-8633 (JP)
(74) Representative: Hoffmann Eitle
(86) International application number: PCT/JP2015/076109
(87) International publication number: WO 2016/056356

(57) **Abstract**

An object of the present invention is to provide a composition for external application containing S-flurbiprofen and mentha oil as a skin penetration enhancer, in which a time-dependent decrease in the content of S-flurbiprofen is suppressed to improve the storage stability of S-flurbiprofen.

To attain this object, at least one member selected from the group consisting of an ester having two medium-chain fatty acids bound to propylene glycol and an ester having three medium-chain fatty acids bound to glycerin is incorporated in a composition for external application containing S-flurbiprofen and mentha oil as a skin penetration enhancer.

## Description

### TECHNICAL FIELD

The present invention relates to compositions for external application that contain S-flurbiprofen and mentha oil, i.e. peppermint oil, and it has applicability in the field of pharmaceuticals.

### BACKGROUND ART

Flurbiprofen having anti-inflammatory and analgesic actions are generally used as an active ingredient in compositions for external application.

Flurbiprofen is 2-(2-fluoro-4-biphenylyl)propionic acid as a racemate and its active S form, S-flurbiprofen, as obtained by optical resolution far excels the racemate in anti-inflammatory and analgesic actions (Patent Document 1).

As a composition for external application that contains S-flurbiprofen, Patent Document 1 has proposed one that contains mentha oil and Sefsol as skin penetration enhancers. However, there has been a room for further improvement in this composition for external application since S-flurbiprofen undergoes chemical reactions and the contents of the active ingredients are shown to decrease over time upon storage.

Also known is a dentifrice composition that is to be filled into a container having the innermost synthetic resin layer and which contains arylpropionic acid based non-steroidal anti-inflammatory agents (medicinal agents) including flurbiprofen (Patent Document 2). The medicinal agents in this dentifrice composition have such an extremely high level of affinity for synthetic resins that they may adsorb on or permeate through the inner surface of the container during storage, so in order to prevent the concentrations of the medicinal agents from decreasing over time, a nonionic surfactant is contained in a specified amount and so are a propylene glycol fatty acid ester and the like. Patent Document 2 relates to a dentifrice composition and neither discloses nor suggests a composition for external application that contains mentha oil as a skin penetration enhancer.

### CITATION LIST

### PATENT LITERATURE

Patent Document 1: JP 08-119859 A
Patent Document 2: JP 07-267838 A

### SUMMARY OF INVENTION

### TECHNICAL PROBLEM

An object of the present invention is to provide a composition for external application containing S-flurbiprofen and mentha oil as a skin penetration enhancer, in which a time-dependent decrease in the content of S-flurbiprofen is suppressed to ensure that S-flurbiprofen is improved in storage stability. Another object of the present invention is to provide a non-aqueous composition for external application containing S-flurbiprofen and mentha oil as a skin penetration enhancer, in which a time-dependent decrease in the content of S-flurbiprofen is suppressed.

### SOLUTION TO PROBLEM

The present inventors conducted an intensive study on a composition for external application containing S-flurbiprofen and mentha oil as a skin penetration enhancer and found as a result that only when a medium-chain fatty acid ester of a certain polyhydric alcohol, namely, at least one member selected from the group consisting of an ester having two medium-chain fatty acids bound to propylene glycol and an ester having three medium-chain fatty acids bound to glycerin, was incorporated, a time-dependent decrease in the content of S-flurbiprofen could be suppressed and the S-flurbiprofen would remain stable even for a prolonged period of storage. This finding has led to the accomplishment of the present invention.

Briefly, the present invention provides:
(1) A composition for external application comprising S-flurbiprofen or a pharmaceutically acceptable salt thereof, mentha oil, and at least one member selected from the group consisting of an ester having two medium-chain fatty acids bound to propylene glycol and an ester having three medium-chain fatty acids bound to glycerin.
(2) The composition as recited in (1) wherein the ester having two medium-chain fatty acids bound to propylene glycol is propylene glycol dicaprylate.
(3) The composition as recited in (1) wherein the ester having three medium-chain fatty acids bound to glycerin is glycerin tricaprate.
(4) The composition as recited in any one of (1) to (3) wherein the at least one member selected from the group consisting of an ester having two medium-chain fatty acids bound to propylene glycol and an ester having three medium-chain fatty acids bound to glycerin is incorporated in an amount 0.1 to 10 times the weight of the S-flurbiprofen or pharmaceutically acceptable salt thereof.
(5) The composition as recited in any one of (1) to (3) wherein the at least one member selected from the group consisting of an ester having two medium-chain fatty acids bound to propylene glycol and an ester having three medium-chain fatty acids bound to glycerin is incorporated in an amount 0.45 to 10 times the weight of the S-flurbiprofen or pharmaceutically acceptable salt thereof.
(6) The composition as recited in any one of (1) to (5) which is a non-aqueous composition.
(7) The composition as recited in (6) which is a tape.

### ADVANTAGEOUS EFFECTS OF INVENTION

According to the present invention, it has become possible to provide a composition for external application containing S-flurbiprofen and mentha oil as a skin penetration enhancer, characterized in that a time-dependent decrease in the content of S-flurbiprofen is suppressed and the S-flurbiprofen will remain stable even for a prolonged period of storage. Especially, in a non-aqueous composition for external application containing S-flurbiprofen and mentha oil as a skin penetration enhancer, it has become possible to suppress a time-dependent decrease in the content of S-flurbiprofen.

### BRIEF DESCRIPTION OF DRAWINGS

Figure 1 is a graph showing how the percent residue of S-flurbiprofen changed over time when samples of Examples 1 and 2 as well as Comparative Examples 1 to 6 were stored under the condition of 50°C.
Figure 2 is a graph showing how the percent residue of S-flurbiprofen changed over time when samples of Examples 1 and 2 as well as Comparative Examples 1 to 6 were stored under the condition of 65°C.
Figure 3 is a graph showing how the percent residue of S-flurbiprofen changed over time when samples of Examples 1 and 3 to 6 were stored under the condition of 50°C.
Figure 4 is a graph showing how the percent residue of S-flurbiprofen changed over time when samples of Examples 1 and 3 to 6 were stored under the condition of 65°C.

### DESCRIPTION OF EMBODIMENTS

Hereinafter, modes of the present invention are described in detail.

The present invention provides a composition for external application comprising S-flurbiprofen or a pharmaceutically acceptable salt thereof, mentha oil, and at least one member selected from the group consisting of an ester having two medium-chain fatty acids bound to propylene glycol and an ester having three medium-chain fatty acids bound to glycerin. The term "medium-chain fatty acids" as used herein refers to saturated fatty acids having 8-12 carbon atoms, preferably caprylic acid, capric acid, etc.

The ester to be used in the present invention that has two medium-chain fatty acids bound to propylene glycol may be exemplified by propylene glycol dicaprylate, propylene glycol di(caprylate/caprate), propylene glycol dicaprate, propylene glycol dilaurate, etc. Among these, propylene glycol dicaprylate is particularly advantageous.

The ester to be used in the present invention that has three medium-chain fatty acids bound to glycerin may be exemplified by glycerin tricaprylate, glycerin triisooctanoate, glycerin tri(caprylate/caprate), glycerin tricaprate, glycerin trilaurate, etc. Among these, glycerin tricaprate is particularly advantageous.

In the present invention, the ester that has two medium-chain fatty acids bound to propylene glycol or the ester that has three medium-chain fatty acids bound to glycerin is preferably incorporated in amounts 0.1 to 10 times, more preferably 0.45 to 10 times, and even more preferably 0.45 to 3.5 times the weight of the S-flurbiprofen or pharmaceutically acceptable salt thereof. If either or both esters are incorporated in amounts less than a tenth of the weight of the S-flurbiprofen or pharmaceutically acceptable salt thereof, its effectiveness in suppressing a time-dependent decrease of S-flurbiprofen is impaired, whereas if the ester's amount is more than 10 times the weight of the S-flurbiprofen or pharmaceutically acceptable salt thereof, the product usability is impaired.

The "S-flurbiprofen or pharmaceutically acceptable salt thereof" to be used in the present invention can be obtained by the methods described in JP 62-6536 B.

The "S-flurbiprofen or pharmaceutically acceptable salt thereof" is preferably S-flurbiprofen.

The "pharmaceutically acceptable salt" is preferably exemplified by a sodium salt and a potassium salt.

The content of S-flurbiprofen or a pharmaceutically acceptable salt thereof is typically from 0.1 to 10 wt%, preferably from 0.5 to 5 wt%, of the total amount of the composition for external application.

The mentha oil to be used in the present invention is an essential oil produced by the following process: the part above ground of *Mentha arvensis Linne var. piperascens Milinvaud (Labiatae)* is subjected to steam distillation and the resulting extract is cooled and freed of the solids content. The mentha oil contains 30.0% or more menthol (C₁₀H₂₀O) and its major components are *l*-menthol, menthone, pinene, camphene, mentenone, limonene, sesquiterpene, etc.

The content of mentha oil is typically from 0.1 to 10 wt%, preferably from 0.5 to 5 wt%, of the total amount of the composition for external application.

The term "non-aqueous" as used herein refers to a system to which no water has been added but incidental moisture is permissible.

The term "storage stability" as used herein refers to such a property of the composition for external application that S-flurbiprofen remains chemically stable when it is stored for an extended period of time.

The composition for external application of the present invention may be provided as a variety of formulations for external application such as tapes (also known as plasters), cataplasms, ointments, creams, lotions, etc. Preferred dosage forms are non-aqueous formulations for external application, such as tapes and ointments, with tapes being particularly preferred. These formulations can be prepared using additives and bases as appropriate for the respective dosage forms in accordance with the usual procedures described in the Japanese Pharmacopoeia (abbreviated as JP) and other literature.

Patches such as tapes and cataplasms can be obtained by a process comprising the steps of preparing an adhesive comprising S-flurbiprofen, mentha oil, and at least one member selected from the group consisting of an ester having two medium-chain fatty acids bound to propylene glycol and an ester having three medium-chain fatty acids bound to glycerin, and making a laminate of a backing layer, an adhesive layer and a peelable liner placed in that order. As will be described later in detail, the base of the adhesive for tapes may optionally contain a tackifier, a softening agent, a solubilizer, etc. As the base of the adhesive for cataplasms, sodium polyacrylate or carboxymethylcellulose sodium (hereinafter abbreviated as CMC-Na) can be used and may optionally contain a tackifier, a thickener, a crosslinker, etc.

The base of the adhesive for tapes may be exemplified by styrene/isoprene/styrene block copolymers (SIS), styrene/butadiene/styrene block copolymers (SBS), styrene/ethylene/butylene/styrene block copolymers (SEBS), and styrene/ethylene/propylene/styrene block copolymers (SEPS), etc., with styrene/isoprene/styrene block copolymers (SIS) being preferred. The preferred SIS is available on the market under trade names such as Kraton D1161JP (product of Kraton Polymers) or Cariflex TR-1107 and TR-1117 (products of Shell Chemicals). The content of the base in the adhesive layer is preferably from 1 to 50 wt%, more preferably from 5 to 40 wt%.

The tackifier may be exemplified by rosin ester resins, polyterpene resins, terpene phenol resins, petroleum resins, etc.; among these, rosin ester resins, in particular, those which have been hydrogenated after removal of low-boiling fractions (e.g. ESTER GUM HG produced by Arakawa Chemical Industries, Ltd.) are preferred. Polyterpene resins include YS-Resin produced by Yasuhara Chemical; terpene phenol resins include YS POLYSTAR also produced by Yasuhara Chemical; petroleum resins that can be used include QUINTONE produced by ZEON CORPORATION, ARCON produced by Arakawa Chemical Industries, Ltd. and ESCOREZ produced by Exxon Mobil. The content of the tackifier in the adhesive layer is preferably from 5 to 50 wt%, with the range of 5 to 30 wt% being particularly preferred.

The softening agent may be exemplified by liquid paraffin, etc.

The base of the adhesive may further contain any other additives such as antioxidants and rubber aging preventing agents. Exemplary rubber aging preventing agents include p,p'-diaminodiphenylmethane, aldole-α-naphthylamine, phenyl-α-naphthylamine, phenyl-β-naphthylamine, N,N'-diphenyl-p-phenyleneamine, N,N'-di-β-naphthyl-p-phenylenediamine, 1,2-dihydro-2,2,4-trimethylquinoline, 2,6-di-tert-butyl-p-cresol, 2,5-di-tert-butyl-hydroquinone, hydroquinone-monobenzylether, 4,4'-dihydroxy-diphenylcyclohexanone, etc., and one or more of these compounds may be incorporated in the base of the adhesive.

It should be noted here that "known aging preventing agents" as mentioned in paragraph 0011 of Patent Document 1 encompass antioxidants.

Patches can be prepared by spreading the aforementioned adhesive layer over a flexible backing layer. The backing here mentioned may be flexible sheeting of any type that does not allow the adhesive in the adhesive layer to permeate through it to reach the opposite side. Specific examples of the sheeting that can be used include woven fabrics, nonwoven fabrics, etc. A suitable thickness of the backing is typically about 1000 µm or less, preferably in the range of 30 to 700 µm.

The aforementioned peelable liner may be of any type that covers the adhesive layer until just before use of the patches and which can be stripped away just before use, and it can be made of the following materials that are processed into a film form: polyesters such as polyethylene terephthalate and polyethylene naphthalate; polyolefins such as polyethylene and polypropylene; polyvinyl chloride; polyvinylidene chloride; cellulose such as paper or derivatives thereof; nylon, aluminum, etc. In order to ensure easy separation from the adhesive layer, the peelable liner is preferably such that it has been surface coated (to become peelable) with a release agent such as silicone or a fluororesin (e.g. Teflon^{®}).

To prepare ointments, S-flurbiprofen, mentha oil, and at least one member selected from the group consisting of an ester having two medium-chain fatty acids bound to propylene glycol and an ester having three medium-chain fatty acids bound to glycerin are dissolved or dispersed in a base such as white petrolatum or macrogol and the resulting solution or dispersion is slowly cooled to room temperature.

To prepare creams, S-flurbiprofen, mentha oil, at least one member selected from the group consisting of an ester having two medium-chain fatty acids bound to propylene glycol and an ester having three medium-chain fatty acids bound to glycerin, and a surfactant are added to an oil phase in which an oil or fat component has been dissolved, and the mixture is degassed and warmed; to the resulting mixture, a warmed aqueous phase is added and high-speed stirring (emulsification) is performed, followed by slow cooling to room temperature, whereby a cream is prepared.

To prepare lotions, S-flurbiprofen, mentha oil, and at least one member selected from the group consisting of an ester having two medium-chain fatty acids bound to propylene glycol and an ester having three medium-chain fatty acids bound to glycerin are dissolved or dispersed in water, ethanol, a polyhydric alcohol or a liquid mixture thereof.

In addition to the above-mentioned components, other substances may be incorporated in those external preparations within quantitative and qualitative ranges that will not impair the effects of the present invention and they include polyhydric alcohols, oils, anionic surfactants, cationic surfactants, amphoteric surfactants, polymers such as cationic polymers, anionic polymers, nonionic polymers, and amphoteric polymers, as well as polysaccharides, chelating agents, antioxidants, UV absorbers, antiseptics, drug substances such as vitamins, scents and so forth.

Polyhydric alcohols include propylene glycol, dipropylene glycol, 1,3-butylene glycol, glycerin, polyethylene glycols, etc.

Nonionic surfactants include sorbitan fatty acid esters such as sorbitan monoleate, glycerin fatty acid esters such as glycerin monostearate, propylene glycol fatty acid esters such as propylene glycol monocaprylate, hardened castor oil derivatives, lipophilic nonionic surfactants such as glycerin alkyl ethers, polyoxyethylene (hereinafter abbreviated as POE) sorbitan fatty acid esters such as POE sorbitan monolaurate, POE glycerin fatty acid esters such as POE glycerin monostearate, POE fatty acid esters such as POE monooleate, POE alkyl phenyl ethers such as POE stearyl ether, pluronics such as Pluronic®, POE/POP alkyl ethers such as POE/POP cetyl ether, tetraPOE/tetraPOP ethylenediamine condensates such as Tetronic®, POE hardened castor oil, POE hardened castor oil derivatives such as POE hardened castor oil monoisostearate, glucose fatty acid esters, etc.

Anionic surfactants include pure soap, fatty acid soaps such as sodium laurate, higher alkyl sulfate ester salts such as sodium lauryl sulfate, alkyl ether sulfate ester salts such as sodium POE lauryl sulfate, phosphate ester salts such as sodium POE oleyl ether phosphate, POE alkylallyl ether carboxylate salts, higher fatty acid ester sulfonate salts, etc.

Amphoteric surfactants include imidazoline-based amphoteric surfactants such as 2-cocoyl-2-imidazolinium hydroxide-1-carboxyethyloxy disodium salt, betaine-based surfactants such as sulfobetaine, amino acid salts such as N-lauryl-β-alanine, and so forth.

Cationic polymers include poly(dimethyldiallylammonium halide) type cationic polymers which are sold under several trade names including Merquat 100 which is available from Merck & Co., Inc., USA.

Anionic polymers include xanthan gum, carrageenan, sodium alginate, gum arabic, pectin, carboxyvinyl polymers, etc.

Nonionic polymers include polyvinylpyrrolidone, vinylpyrrolidone/vinyl acetate copolymer, vinylpyrrolidone/vinyl acetate/acrylaminoacrylate copolymer, hydroxyethyl cellulose, hydroxypropyl cellulose, hydroxypropyl methyl cellulose, methyl cellulose, dextrin, galactan, pullulan, etc.

Amphoteric polymers include dialkylamine ethyl acrylates, dialkylamino ethyl methacrylates, diacetone acrylamides, etc. as copolymerized with acrylic acid, acrylic acid alkyl esters, methacrylic acid, methacrylic acid alkyl esters, etc., followed by rendering the copolymerization product amphoteric with acetic acid halides, etc.; among these polymers is the commercial product sold under the trade name YUKAFORMER AM-75 from Mitsubishi Chemical Corporation.

Polysaccharides include mucopolysaccharides such as hyaluronic acid, chondroitin sulfate, and salts thereof.

Ultraviolet absorbers include, for example, benzoic acid-based compounds such as para-aminobenzoic acid, cinnamate-based compounds such as octyl methoxycinnamate, benzophenone-based compounds such as 2,4-dihydroxybenzophenone.

Antiseptics include those which are commonly used in pharmaceutics, quasi-drugs, and cosmetics, as exemplified by dibutyl hydroxytoluene, paraoxybenzoic acid alkyl esters, sodium benzoate, and potassium sorbate.

Drug substances such as vitamins include those which are commonly used in pharmaceutics, quasi-drugs, and cosmetics, as exemplified by vitamins such as vitamins C and vitamins E, placental extract, a variety of amino acids, crude drugs, and anti-inflammatory agents.

### FORMULATION EXAMPLES

As formulation examples of the present invention, a tape, a cataplasm, an ointment and a lotion may be given and their respective recipes are listed in Table A below.

**[Table A]**

| Ingredients | Concentrations (wt%) in | | | |
|---|---|---|---|---|
| | Tape | Cataplasm | Ointment | Lotion |
| S-flurbiprofen | 2 | 0.2 | 2.3 | 3 |
| Mentha oil | 3 | 0.3 | 2.1 | 2.7 |
| Propylene glycol dicaprylate | 3 | 0.3 | 4.2 | 5.4 |
| SIS | 35 | - | - | - |
| Polyisobutylene | 15 | - | - | - |
| Hydrogenated rosin ester resin | 35 | - | - | - |
| Liquid paraffin | 7 | - | - | - |
| CMC-Na | - | 4 | - | - |
| Sodium polyacrylate | - | 5 | - | - |
| Glycerin | - | 25 | - | - |
| Kaolin | - | 10 | - | - |
| Polyacrylic acid alkyl ester | - | 6 | - | - |
| White petrolatum | - | - | 91.4 | - |
| 1,3-Butylene glycol | - | - | - | 20 |
| Absolute ethanol | - | - | - | 50 |
| Water | - | 49.2 | - | 18.7 |
| Dibutylhydroxytoluene | q. s. | q. s. | q. s. | q. s. |
| 2-Mercaptobenzimidazole | q. s. | q. s. | q. s. | q. s. |
| Sodium hydroxide | - | - | - | q. s. |

The tape may be prepared from the ingredients listed in Table B below. Typically, the active ingredients and additives are kneaded, optionally under warming conditions, to prepare an adhesive, which is spread and cut into a predetermined size, then adhered to a backing, protected with a liner material such as a film, and the resulting formulation is packed into a wrap bag.

**[Table B]**

| Description | Ingredients | Amount incorporated | Concentration |
|---|---|---|---|
| Active ingredient | S-flurbiprofen | 40.0 mg | 2.31% |
| Active ingredient | Mentha oil (JP) | 36.2 mg | 2.09% |
| Softening agent | Liquid paraffin (JP) | q. s. | - |
| Base | Styrene/isoprene/styrene block copolymer | q. s. | - |
| Base | Polyisobutylene | q. s. | - |
| Tackifier | Ester gum | q. s. | - |
| Solvent promoter | Propylene glycol dicaprylate | q. s. | - |
| Antioxidant | Dibutyl hydroxytoluene | q. s. | - |
| Others | | q. s. | - |
| | Total (adhesive's weight) | 1730 mg | 100% |
| Backing | Nonwoven fabric | 140 cm² | |
| Liner | Polyester film | 140 cm² | |
| | Container and closure system | 7 sheets/Al bag | |

### EXAMPLES

Hereinafter, the present invention will be described in greater detail based on Examples, to which the present invention is by no means limited.

### [Method of Preparation]

In accordance with the recipe shown in Table 1 below, S-flurbiprofen, mentha oil and polyhydric alcohol medium-chain fatty acid esters were aliquoted in glass bottles, which were heated on a 65°C water bath until a solution formed; in this way, respective samples were prepared.

### [Test Method]

Each sample was distributed among glass test tubes and stored in a thermostated vessel of 50°C or 65°C. Before the start of storage and after the passage of 1, 2 and 3 months of storage, samples were collected and the percent residue of S-flurbiprofen in each sample was measured to evaluate its stability.

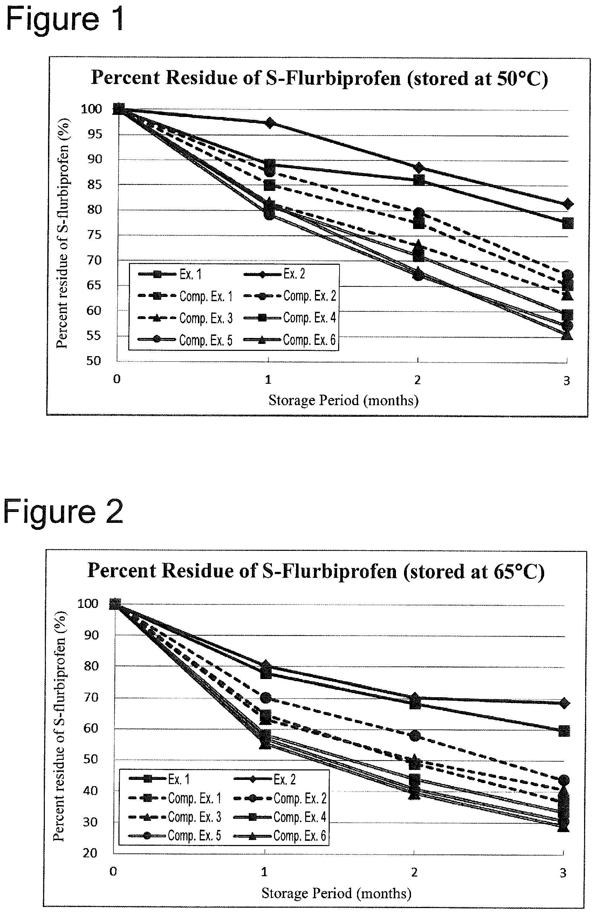

Table 1 shows the recipes of the samples and how the percent residue of S-flurbiprofen changed over time when they were stored under the conditions mentioned above. The data in Table 1 compare the time-dependent profiles of the percent residue of S-flurbiprofen for the cases where the active ingredients were mixed with a prescribed amount of the various polyhydric alcohol medium-chain fatty acid esters listed in the table. The time-dependent profiles of the percent residue of S-flurbiprofen are also depicted in Figs. 1 and 2.

**[Table 1]**

| Ingredients | | | Example | | Comparative Example | | | | | |
|---|---|---|---|---|---|---|---|---|---|---|
| | | | 1 | 2 | 1 | 2 | 3 | 4 | 5 | 6 |
| | | | Concentration (wt%) | | | | | | | |
| S-flurbiprofen | | | 27.36 | 27.36 | 27.36 | 27.36 | 27.36 | 27.36 | 27.36 | 27.36 |
| Mentha oil | | | 24.76 | 24.76 | 24.76 | 24.76 | 24.76 | 24.76 | 24.76 | 24.76 |
| Propylene glycol dicaprylate | | | 47.88 | - | - | - | - | - | - | - |
| Glycerin tricaprylate | | | - | 47.88 | - | - | - | - | - | - |
| Propylene glycol monocaprylate | | | - | - | 47.88 | | - | - | - | - |
| Sorbitan monolaurate | | | - | - | - | 47.88 | - | - | - | - |
| Ethylene glycol monolaurate | | | - | - | - | - | 47.88 | - | - | - |
| Decaglyceryl monolaurate | | | - | - | - | - | - | 47.88 | - | - |
| Hexaglyceryl monolaurate | | | - | - | - | - | - | - | 47.88 | - |
| Glycerin monocaprylate | | | - | - | - | - | - | - | - | 47.88 |
| Total | | | 100 | 100 | 100 | 100 | 100 | 100 | 100 | 100 |
| Ratio of polyhydric alcohol medium-chain fatty acid ester to S-flurbiprofen | | | 1.75 | 1.75 | 1.75 | 1.75 | 1.75 | 1.75 | 1.75 | 1.75 |
| Percent residue of S-flurbiprofen (%) | Stored at 50°C | After 1 month | 89.1 | 97.4 | 85.1 | 87.7 | 81.4 | 80.9 | 79.1 | 81.3 |
| | | After 2 months | 86.1 | 88.6 | 77.5 | 79.6 | 73.1 | 71.0 | 67.2 | 67.8 |
| | | After 3 months | 77.7 | 81.4 | 65.4 | 67.4 | 63.5 | 59.5 | 57.3 | 55.6 |
| | Stored at 65°C | After 1 month | 77.9 | 80.3 | 64.6 | 70.1 | 63.2 | 58.1 | 56.8 | 55.4 |
| | | After 2 months | 68.4 | 70.2 | 49.0 | 58.0 | 50.2 | 44.0 | 40.8 | 39.5 |
| | | After 3 months | 59.8 | 68.7 | 37.0 | 43.9 | 40.8 | 33.7 | 30.8 | 29.2 |

The polyhydric alcohol medium-chain fatty acid ester contained in the samples of Example 1 was propylene glycol dicaprylate having two medium-chain fatty acids bound to propylene glycol, whereas the polyhydric alcohol medium-chain fatty acid ester contained in the samples of Example 2 was glycerin tricaprylate having three medium-chain fatty acids bound to glycerin. Compared to the samples of Comparative Examples 1-6 containing polyhydric alcohol medium-chain fatty acid esters in which only one medium-chain fatty acid bound to propylene glycol or glycerin, the samples of Examples 1 and 2 maintained significantly high percent residues of S-flurbiprofen even after the storage of 3 months, experiencing only gradual decreases in the percent residue of S-flurbiprofen.

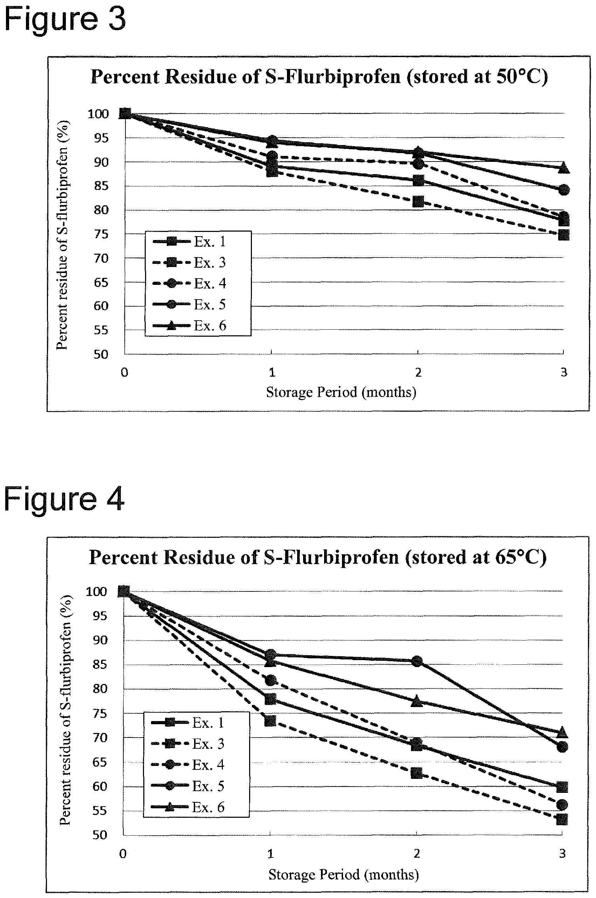

The propylene glycol dicaprylate which caused only a gradual decrease in the percent residue of S-flurbiprofen was further investigated for the cases where its content was varied from 0.45 to 3.50 times the weight of S-flurbiprofen. Table 2 shows the recipes of the samples and how the percent residue of S-flurbiprofen changed over time when they were stored under the conditions mentioned above. The time-dependent profiles of the percent residue of S-flurbiprofen are also depicted in Figs. 3 and 4.

**[Table 2]**

| Ingredients | | | Example | | | | |
|---|---|---|---|---|---|---|---|
| | | | 3 | 4 | 1 | 5 | 6 |
| | | | Concentration (wt%) | | | | |
| S-flurbiprofen | | | 42.46 | 35.97 | 27.36 | 22.08 | 18.50 |
| Mentha oil | | | 38.43 | 32.56 | 24.76 | 19.97 | 16.74 |
| Propylene glycol dicaprylate | | | 19.11 | 31.47 | 47.88 | 57.95 | 64.76 |
| Total | | | 100 | 100 | 100 | 100 | 100 |
| Ratio of propylene glycol dicaprylate to S-flurbiprofen | | | 0.45 | 0.87 | 1.75 | 2.62 | 3.50 |
| Percent residue of S-flurbiprofen (%) | Stored at 50°C | After 1 month | 88.0 | 91.1 | 89.1 | 94.4 | 94.0 |
| | | After 2 months | 81.7 | 89.6 | 86.1 | 91.8 | 92.0 |
| | | After 3 months | 74.7 | 78.5 | 77.7 | 84.1 | 88.7 |
| | Stored at 65°C | After 1 month | 73.5 | 81.8 | 77.9 | 87.0 | 85.7 |
| | | After 2 months | 62.7 | 68.9 | 68.4 | 85.7 | 77.5 |
| | | After 3 months | 53.2 | 56.3 | 59.8 | 68.1 | 71.0 |

The samples of Examples 1 and 3 to 6 incorporating varying proportions of propylene glycol dicaprylate relative to S-flurbiprofen maintained significantly high percent residues of S-flurbiprofen at all of the proportions tested and even after the storage of 3 months, as compared with the comparative samples. Furthermore, as the above-defined relative proportions of propylene glycol dicaprylate became larger, the reduction of residual percentage of S-flurbiprofen was suppressed.

### INDUSTRIAL APPLICABILITY

According to the present invention, at least one member selected from the group consisting of an ester having two medium-chain fatty acids bound to propylene glycol and an ester having three medium-chain fatty acids bound to glycerin is incorporated in a composition for external application containing S-flurbiprofen and mentha oil as a skin penetration enhancer, whereby it has become possible to provide a product in which a time-dependent decrease in the content of S-flurbiprofen is suppressed to improve the storage stability of S-flurbiprofen. Especially, in a non-aqueous composition for external application containing S-flurbiprofen and mentha oil as a skin penetration enhancer, it has become possible to suppress a time-dependent decrease in the content of S-flurbiprofen.

## Claims

1. A composition for external application comprising S-flurbiprofen or a pharmaceutically acceptable salt thereof, mentha oil, and at least one member selected from the group consisting of an ester having two medium-chain fatty acids bound to propylene glycol and an ester having three medium-chain fatty acids bound to glycerin.

2. The composition according to claim 1 wherein the ester having two medium-chain fatty acids bound to propylene glycol is propylene glycol dicaprylate.

3. The composition according to claim 1 wherein the ester having three medium-chain fatty acids bound to glycerin is glycerin tricaprate.

4. The composition according to any one of claims 1 to 3 wherein the at least one member selected from the group consisting of an ester having two medium-chain fatty acids bound to propylene glycol and an ester having three medium-chain fatty acids bound to glycerin is incorporated in an amount which is 0.1 to 10 times the weight of the S-flurbiprofen or pharmaceutically acceptable salt thereof.

5. The composition according to any one of claims 1 to 3 wherein the at least one member selected from the group consisting of an ester having two medium-chain fatty acids bound to propylene glycol and an ester having three medium-chain fatty acids bound to glycerin is incorporated in an amount which is 0.45 to 10 times the weight of the S-flurbiprofen or pharmaceutically acceptable salt thereof.

6. The composition according to any one of claims 1 to 5 which is a non-aqueous composition.

7. The composition according to claim 6 which is a tape.
